(19) Europäisches Patentamt
European Patent Office
Office européen des brevets

(11) EP 0 911 018 B1

(12) **EUROPÄISCHE PATENTSCHRIFT**

(45) Veröffentlichungstag und Bekanntmachung des Hinweises auf die Patenterteilung:
**28.05.2003 Patentblatt 2003/22**

(51) Int Cl.[7]: **A61K 7/06**, A61K 7/50

(21) Anmeldenummer: **98118850.1**

(22) Anmeldetag: **06.10.1998**

# (54) Verwendung von kationischen Copolymerisaten aus ungesättigten Säuren und N-Vinylimidazoliumsalzen in haarkosmetischen Zubereitungen

Use of cationic copolymers of unsaturated acids and n-vinylimidazolium salts in hair cosmetic compositions

Utilisation de copolymers cationiques d'acides insatures et de sels de n-vinylimidazol dans des compositions cosmétiques capilllaires

(84) Benannte Vertragsstaaten:
**DE ES FR GB IT**

(30) Priorität: **16.10.1997 DE 19745637**

(43) Veröffentlichungstag der Anmeldung:
**28.04.1999 Patentblatt 1999/17**

(73) Patentinhaber: **BASF AKTIENGESELLSCHAFT 67056 Ludwigshafen (DE)**

(72) Erfinder:
- **Dieing, Reinhold Dr. 67105 Schifferstadt (DE)**
- **Hössel, Peter Dr. 67105 Schifferstadt (DE)**
- **Sanner, Axel Dr. 67227 Frankenthal (DE)**

(56) Entgegenhaltungen:
**EP-A- 0 100 890** **EP-A- 0 715 843**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 911 018 B1

Printed by Jouve, 75001 PARIS (FR)

## Beschreibung

Die vorliegende Erfindung betrifft die Verwendung von kationischen Polymeren, erhältlich durch radikalisch initiierte Copolymerisation von Monomergemischen aus

(a) 60 bis 99 Gew.-% eines 1-Vinylimidazols oder eines quaternisierten 1-Vinylimidazols,

(b) 1 bis 40 Gew.-% einer ungesättigten Säure oder deren Salzen,

(c) 0 bis 30 Gew.-% eines weiteren radikalisch copolymerisierbaren Monomeren

und gegebenenfalls anschließende Quaternisierung des Polymeren als Wirkstoffe in haarkosmetischen Zubereitungen, insbesondere als Konditioniermittel in Shampoos.

Haarshampoos haben die Aufgabe, das Haar von Verunreinigungen zu befreien. Moderne Shampoos erfüllen neben dieser Reinigungswirkung zusätzlich konditionierende Funktionen. Die konditionierende Wirkung wird durch den Gehalt von Konditioniermitteln in der Shampoo-Zusammensetzung erreicht.
Beispiele für Konditioniermittel, die Anwendung in Shampoos finden, sind vor allem Silikone und kationische Polymere. Die Silikone haben den Nachteil, daß sie meist wasserunlöslich sind und die Shampoo-Formulierung durch Dispergiermittel stabilisiert werden muß. Diese Zusätze sind unerwünscht. Ferner zeigen Silikone einen starken Anreicherungseffekt, das heißt, die Silikone ziehen auf das Haar auf und werden durch das Waschen der Haare nicht vollständig entfernt. Nach einiger Zeit fühlen sich die Haare unangenehm beschwert an.

Die kationischen Polymere, die als Konditioniermittel in Shampoos Verwendung finden, wie kationische Cellulosederivate, bilden aber mit den Aniontensiden der Shampoo-Formulierung Tensid-Polymerkomplexe, die bei hoher Ladungsdichte der Polymere wasserunlöslich sind. Deshalb werden üblicherweise kationische Polymere mit niedriger Ladungsdichte eingesetzt, damit sie in der Formulierung löslich sind.

Da jedoch kationische Polymere mit hoher Ladungsdichte eine größere Affinität zum Haar zeigen, ist es wünschenswert, hochgeladene Polymere in Shampoos einzusetzen. Allerdings sind dann die Tensid-Polymer-Komplexe in der Formulierung unlöslich. Die Formulierung muß durch Zusatz von Dispergierhilfsmitteln stabilisiert werden.

So wird in WO 94/06403 die Verwendung von u.a. Copolymeren aus N-Vinylpyrrolidon und 3-Methyl-1-vinylimidazoliumsalzen mit hoher Ladungsdichte in Kombination mit weiteren wasserunlöslichen Konditioniermitteln in Shampoo-Formulierungen vorgeschlagen. Zur Stabilisierung der Formulierungen werden dementsprechend Dispergiermittel eingesetzt. Ferner werden in WO 94/06409 und US 5580494 Shampoo-Zusammensetzungen auf Basis eines Alpha-Olefinsulfonates als Detergens und eines kationischen Polymers mit hoher Ladungsdichte, z.B. Copolymeren aus N-Vinylpyrrolidon und 3-Methyl-1-vinylimidazoliumsalzen als Konditioniermittel, beschrieben. Auch hier müssen zur Stabilisierung der Formulierungen Dispergierhilfsmittel zugesetzt werden.

Ferner ist aus EP-A 246 580 bekannt, quaternisierte Vinylimidazol-Copolymerisate mit verschiedenen weiteren Monomeren, jedoch ohne polymerisierbare ungesättigte Säuren, als Haarkonditioniermittel zu verwenden. Die dort beschriebenen Polymere haben den Nachteil, daß sie im Falle eines geringen Anteils der quaternisierten Vinylimidazol-Monomeren in Gegenwart von Aniontensiden eine geringe Wirkung bzw. im Falle eines hohen Anteils des quaternisierten Vinylimidazols keine stabilen Dispersionen bilden.

Es war daher die Aufgabe der vorliegenden Erfindung, kationische Polymere mit hoher Ladungsdichte zu finden, die in Shampoo-Formulierungen mit Aniontensiden ohne zusätzliche Dispergierhilfsmittel formuliert werden können.

Diese Aufgabe wurde gelöst durch Verwendung von kationischen Copolymerisaten, erhältlich durch radikalisch initiierte Copolymerisation von

(a) 60 bis 99 Gew.-%, vorzugsweise 65 bis 95 Gew.-% und, besonders bevorzugt 70 bis 90 Gew.-% eines gegebenenfalls substituierten 1-Vinylimidazols oder eines quaternisierten 1-Vinylimidazols,

(b) 1 bis 40 Gew.-%, vorzugsweise 5 bis 35 Gew.-%, besonders bevorzugt 10 bis 30 Gew.-%, einer eine polymerisierbare Doppelbindung enthaltenden Säure oder deren Salzen und

(c) 0 bis 30 Gew.-%, vorzugsweise 0 bis 20 Gew.-%, besonders bevorzugt 0 bis 10 Gew.-% eines weiteren radikalisch copolymerisierbaren Monomeren

und anschließende Quaternisierung des Polymeren, sofern als Monomeres (a) ein nicht quaternisiertes 1-Vinylimidazol eingesetzt wird, als Wirkstoffe in haarkosmetischen Zubereitungen, insbesondere als Konditioniermittel in Shampoos.

Die Quaternisierung der Polymeren wird dabei in der Regel so durchgeführt, daß eine vollständige Quaternisierung der Vinylimidazol-Einheiten erfolgt. Es ist jedoch auch möglich, nur eine teilweise Quaternisierung z.B. bis zu 80 % und vorzugsweise über 90 % vorzunehmen.

Als Monomer (a) kommen 1-Vinylimidazol oder dessen Derivate der allgemeinen Formel I in Betracht

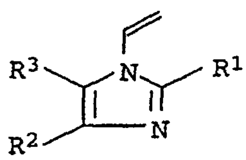

I,

in der $R^1$ bis $R^3$ unabhängig voneinander Wasserstoff, $C_1$-$C_4$-Alkyl oder Phenyl bedeutet.

Bevorzugt sind 1-Vinylimidazol und 2-Methyl-1-vinylimidazol.

Zur Quaternisierung der Verbindungen der allgemeinen Formel I kommen an sich bekannte Methoden zur Anlagerung von Alkyl-, Aralkyl- oder Hydroxyalkylresten in Betracht, beispielsweise die Umsetzung mit Alkylhalogeniden mit 1 bis 24 C-Atomen wie Methylchlorid, Methylbromid, Methyliodid, Ethylchlorid, Ethylbromid, Propylchlorid, Hexylchlorid, Dodecylchlorid, Laurylchlorid und mit Benzylhalogeniden, insbesondere Benzylchlorid und Benzylbromid. Weitere besonders geeignete Quaternierungsmittel sind Dialkylsulfate, insbesondere Dimethylsulfat oder Diethylsulfat. Die Quaternisierung der basischen Monomere oder Polymere kann auch mit Alkylenoxiden wie Ethylenoxid oder Propylenoxid in Gegenwart von Säuren durchgeführt werden.

Bevorzugte Quaternierungsmittel sind: Methylchlorid, Dimethylsulfat oder Diethylsulfat.

Monomere (b) sind z.B. ungesättigte Carbonsäuren mit 2 bis 8 C-Atomen wie Acrylsäure, Methacrylsäure, Dimethylacrylsäure, Ethacrylsäure, Crotonsäure, Itaconsäure, Methylenmalonsäure, Maleinsäure und Maleinsäurehalbester, Fumarsäure und Fumarsäurehalbester. Weiterhin eignen sich ungesättigte Sulfonsäuren, wie Vinylsulfonsäure oder Acrylamidomethylpropansulfonsäure, sowie ungesättigte Phosphonsäuren, beispielsweise Vinylphosphonsäure. Die ungesättigten Säuren können entweder in Form der freien Säuren oder in partiell oder vollständig neutralisierter Form in den Copolymerisaten vorliegen. Die Monomeren werden gegebenenfalls mit Alkalihydroxiden, beispielsweise Natriumhydroxid oder Kaliumhydroxid, Ammoniak oder Aminen neutralisiert.
Bevorzugte Monomere (b) sind Acrylsäure, Methacrylsäure und Acrylamidomethylpropansulfonsäure.

Als Monomere (c) kommen alle Monomere in Betracht, die mit den Monomeren (a) und (b) copolymerisierbar sind. Beispielsweise eignen sich N-Vinyllactame, z.B. N-Vinylpiperidon, N-Vinylpyrrolidon oder N-Vinylcaprolactam, ferner N-Vinylacetamid, N-Methyl-N-vinylacetamid, Acrylamid, Methacrylamid, N,N-Dimethylacrylamid, N-Methylolmethacrylamid, N-Vinylformamid, N-Vinyloxazolidon, N-Vinyltriazol, Hydroxyalkyl (meth)acrylate, z.B. Hydroxyethyl(meth) acrylat und Hydroxypropyl(meth)acrylate, oder Alkylethlenglykol(meth)acrylate mit 1 bis 50 Ethylenglykoleinheiten im Molekül. Weiterhin eignen sich Dialkylaminoalkylacrylate und -methacrylate, beispielsweise Dimethylaminoethylmethacrylat, oder Dialkylaminoalkylacrylamide und -methacrylamide, beispielsweise Dimethylaminopropylmethacrylamid.
Ferner sind $C_1$- bis $C_{24}$-, insbesondere $C_1$- bis $C_{10}$-Alkylester der Acryl- oder Methacrylsäure zu nennen, z.B. Methylacrylat, Methylmethacrylat, Ethylacrylat, Ethylmethacrylat, tert.-Butylacrylat, tert.-Butylmethacrylat, Isobutylacrylat, n-Butylacrylat und Acrylamide wie N-tert.-Butylacrylamid oder N-tert.-Octylacrylamid. Weiterhin können Carbonsäurevinylester, z.B. Vinylacetat oder Vinylpropionat, eingesetzt werden.

Die Monomeren der Gruppen (a) bis (c) können jeweils einzeln oder im Gemisch mit weiteren Monomeren der gleichen Gruppe eingesetzt werden.

Besonders bevorzugt sind Copolymerisate aus

(a) 70 bis 90 Gew.-% 3-Methyl-1-vinylimidazolium-chlorid oder -methylsulfat und
(b) 10 bis 30 Gew.-% Acrylsäure, Methacrylsäure oder Acrylamidomethylpropansulfonsäure oder deren Salzen.

Die Herstellung der Polymerisate kann nach den an sich bekannten Verfahren der radikalisch initiierten Polymerisation, z.B. durch Lösungspolymerisation, Emulsionspolymerisation, Suspensionspolymerisation, Fällungspolymerisation, Umgekehrte Suspensionspolymerisation oder Umgekehrte Emulsionspolymerisation erfolgen, ohne daß die verwendbaren Methoden darauf beschränkt sind.
Vorzugsweise erfolgt die Herstellung als Lösungspolymerisation in Lösungsmitteln wie Wasser, Methanol, Ethanol, Isopropanol oder Mischungen dieser Lösungsmittel. Man wählt die Mengen an Monomeren und Lösungsmitteln zweckmäßigerweise so, daß 15 bis 60 gew.-%ige Lösungen entstehen.

Die Polymerisation erfolgt üblicherweise bei Temperaturen von 20 bis 130°C und bei Normaldruck oder unter Eigendruck.

Als Initiatoren für die radikalische Polymerisation können die hierfür üblichen wasserlöslichen und wasserunlöslichen Peroxo- und/oder Azo-Verbindungen eingesetzt werden, beispielsweise Alkali- oder Ammoniumperoxidisulfate, Dibenzoylperoxid, tert.-Butylperpivalat, tert.-Butyl-per-2-ethylhexanoat, Di-tert.-butylperoxid, tert.-Butylhydroperoxid, Azo-bis-isobutyronitril, Azobis-(2-amidinopropan)dihydrochlorid oder 2,2'-Azo-bis-(2-methylbutyronitril). Geeignet sind auch Initiatormischungen oder Redox-Initiator-Systeme wie Ascorbinsäure/Eisen(II)sulfat/Natriumperoxodisulfat, tert.-Butylhydroperoxid/Natriumsulfit, tert.-Butylhydroperoxid/Natriumhydroxymethansulfinat. Die Initiatoren können in den üblichen Mengen eingesetzt werden, beispielsweise in Mengen von 0,05 bis 5 Gew.-%, bezogen auf die Menge der zu polymerisierenden Monomeren.

Die K-Werte werden nach Fikentscher, Cellulosechemie, Bd. 13, S. 58-64 (1932) bei 25°C 1%ig in 0,5 molarer Kochsalzlösung gemessen und liegen im Bereich von 10 bis 200, vorzugsweise 20 bis 150.

Die erfindungsgemäß zu verwendenden Polymere eignen sich allgemein als Konditionierungsmittel in haarkosmetischen Zubereitungen wie Haarkuren, Haarlotionen, Haarspülungen, Haaremulsionen, Spitzenfluids, Egalisierungsmitteln für Dauerwellen, 'Hot-Oil-Treatment'-Präparate, Conditioner, Festigerlotionen oder Haarsprays.

Je nach Anwendungsgebiet können die haarkosmetischen Zubereitungen als Spray, Schaum, Gel, Gelspray oder Mousse appliziert werden.

Die haarkosmetischen Zubereitungen können neben den erfindungsgemäßen Polymeren und geeigneten Lösungsmitteln wie Wasser oder Wasser/Alkohol-Gemischen noch in der Kosmetik übliche Zusätze wie Emulgatoren, Konservierungsmittel, Parfümöle, Pflegestoffe wie Panthenol, Collagen, Vitamine, Eiweißhydrolysate, Stabilisatoren, pH-Wert-Regulatoren, Farbstoffe und weitere übliche Additive enthalten.

Man kann die erfindungsgemäß zu verwendenden Polymere auch mit herkömmlichen Haarkosmetik-Polymeren abmischen, falls spezielle Eigenschaften eingestellt werden sollen. Als herkömmliche Haarkosmetik-Polymere eignen sich beispielsweise anionische Polymere. Solche anionischen Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe. Besonders geeignete Polymere sind Copolymere aus tert.-Butylacrylat, Ethylacrylat, Methacrylsäure (z. B. Luvimer® 100P, 36D, 30E, MAE), Copolymere aus N-tert.-Butylacrylamid, Ethylacrylat, Acrylsäure (Ultrahold® 8, strong), Copolymere aus Vinylacetat, Crotonsäure, Vinylpropiat (z.B. Luviset® CAP), Maleinsäureanhydridcopolymere, ggf. mit Alkoholen umgesetzt, anionische Polysiloxane, z.B. carboxyfunktionelle, Copolymere aus Vinylpyrrolidon, tert. -Butylacrylat und Methacrylsäure (z.B. Luviskol® VBM).

Ganz besonders bevorzugt werden als anionische Polymere Acrylate mit einer Säurezahl größer gleich 120 und Copolymere aus t-Butylacrylat, Ethylacrylat, Methacrylsäure.

Weitere geeignete Haarkosmetik-Polymere, die zusammen mit den erfindungsgemäß zu verwendenden Polymeren Anwendung finden, sind z.B. kationische Polymere mit der Bezeichnung Polyquaternium nach INCI, z.B. Copolymere aus Vinylpyrrolidon/N-Vinylimidazoliumsalzen (Luviquat® FC, Luviquat® HM, Luviquat® MS), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4- und -10), Acrylamidcopolymere (Polyquaternium-7).

Als weitere Bestandteile in Haarkosmetik-Zubereitungen kommen auch neutrale Polymere in Betracht wie Polyvinylpyrrolidone, Copolymere aus N-Vinylpyrrolidon und Vinylacetat und/oder Vinylpropionat, Polysiloxane, Polyvinylcaprolactam und Copolymere mit N-Vinylpyrrolidon, Polyethylenimine und deren Salze, Polyvinylamine und deren Salze, Cellulosederivate, Polyasparaginsäuresalze und Derivate.

Zur Einstellung bestimmte Eigenschaften können die Zubereitungen zusätzlich in geringen Mengen auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane oder Silikonharze.

Die erfindungsgemäß zu verwendenden Polymerisate eignen sich insbesondere zur Verwendung als Konditioniermittel in Shampoo-Formulierungen. Demgemäß sind auch Gegenstand der Erfindung Shampoo-Formulierungen, die die oben definierten Copolymere enthalten.

In den Shampoo-Formulierungen können alle in Shampoos üblicherweise eingesetzte anionische, neutrale, amphotere oder kationische Tenside verwendet werden. Üblicherweise sind dies anionische Tenside als Basistenside und amphotere und nichtionische Tenside als Cotenside.

Die Shampoo-Formulierungen enthalten in der Regel 2 bis 50 Gew.-% Tenside, bevorzugt 5 bis 40 Gew.-%, besonders bevorzugt 8 bis 30 Gew.-%.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z.B. Natrium-, Kalium-, Magnesium-, Calcium-, sowie Ammonium- und Triethanolamin-Salze. Die Alkylethersulfate, Alkyletherphosphate und Alkylethercarboxylate können zwischen 1 bis 10 Ethylenoxid- oder Propylenoxid-Einheiten, bevorzugt 1 bis 3 Ethylenoxid-Einheiten im Molekül aufweisen. Geeignet sind zum Beispiel Natriumlaurylsulfat, Ammoniumlaurylsulfat, Natriumlaurylethersulfat, Ammoniumlaurylethersulfat, Natriumlaroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder dipropionate. Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampoo-Formulierungen übliche kationische Tenside enthalten, wie quaternäre Ammoniumverbindungen, beispielsweise Cetyltrimethylammoniumchlorid.

Die erfindungsgemäß zu verwendenden Polymere werden üblicherweise in Mengen zwischen 0,01 bis 5 Gew.-% eingesetzt, bevorzugt zwischen 0,05 und 2 Gew.-%, jeweils bezogen auf das fertige Shampoo.

Zusätzlich können auch weitere in Shampoos übliche kationische Polymere eingesetzt werden, so z.B. Copolymere aus Acrylamid und Dimethyldiallylammoniumchlorid (Polyquaternium-7), kationische Cellulosederivate (Polyquaternium-10), Guarhydroxypropyltrimethylammoniumchlorid (INCI: Hydroxypropyl Guar Hydroxypropyltrimonium Chloride), Copolymere aus N-Vinylpyrrolidon und quaternisiertem N-Vinylimidazol (Polyquaternium-16, -44, -46) und andere.

Weiterhin können die Shampoo-Formulierungen Verdicker, wie Kochsalz, PEG-55, Propylene Glycol Oleate oder PEG-120 Methyl Glucose Dioleate, sowie Konservierungsmittel, weitere Wirk- und Hilfsstoffe und Wasser enthalten.

A) Herstellung der Polymere

Beispiel 1

80 g Methacrylsäure und 173 g Wasser wurden gemischt und mit 50 %iger Natronlauge auf einen pH-Wert von 7 eingestellt. Danach wurden 533 g einer 60 %igen wässerigen Lösung von 3-Methyl-1-vinylimidazoliumchlorid zugegeben (Zulauf 1). In einer Rührapparatur mit Stickstoffspülung wurden 70 g Wasser, 100 g von Zulauf 1 und 5 g von Zulauf 2, bestehend aus 3 g 2,2'-Azo-bis(2-amidinopropan)dihydrochlorid und 75 g Wasser, vorgelegt. Man erhitzte auf 60°C und dosierte Zulauf 1 und 2 in jeweils 4 Stunden zu. Danach wurde die Temperatur auf 70°C erhöht und weitere 2 g 2,2'-Azo-bis(2-amidinopropan)dihydrochlorid in 75 ml Wasser zugegeben. Man rührte noch 1 Stunde bei 70°C und erhielt eine viskose Lösung mit einem Feststoffgehalt von 41,6 % und einem K-Wert von 56.

Beispiel 2

100 g Methacrylsäure und 280 g Wasser wurden gemischt und mit 50 %iger Natronlauge auf einen pH-Wert von 7,2 eingestellt. Danach wurden 500 g einer 60 %igen wässerigen Lösung von 3-Methyl-1-vinylimidazoliumchlorid zugegeben (Zulauf 1). In einer Rührapparatur mit Stickstoffspülung wurden 100 g Wasser, 100 g von Zulauf 1 und 5 g von Zulauf 2, bestehend aus 3 g 2,2'-Azo-bis(2-amidinopropan)dihydrochlorid und 60 g Wasser, vorgelegt. Man erhitzte auf 60°C und dosierte Zulauf 1 und 2 in jeweils 4 Stunden zu. Danach wurde die Temperatur auf 70°C erhöht und weitere 2 g 2,2'-Azobis(2-amidinopropan)dihydrochlorid in 75 ml Wasser zugegeben. Man rührte noch 1 Stunde bei 70°C und erhielt eine viskose Lösung mit einem Feststoffgehalt von 41,6 % und einem K-Wert von 56.

Beispiel 3

120 g Methacrylsäure und 320 g Wasser wurden gemischt und mit 50 %iger Natronlauge auf einen pH-Wert von 7 eingestellt. Danach wurden 445 g einer 60 %igen wässerigen Lösung von 3-Methyl-1-vinylimidazoliumchlorid zugegeben (Zulauf 1). In einer Rührapparatur mit Stickstoffspülung wurden 70 g Wasser, 100 g von Zulauf 1 und 5 g von Zulauf 2, bestehend aus 3 g 2,2'-Azo-bis(2-amidinopropan)dihydrochlorid und 75 g Wasser, vorgelegt. Man erhitzte auf 60°C und dosierte Zulauf 1 und 2 in jeweils 4 Stunden zu. Danach wurde die Temperatur auf 70°C erhöht und weitere 2 g 2,2'-Azo-bis (2-amidinopropan)dihydrochlorid in 75 ml Wasser zugegeben. Man rührte noch 1 Stunde bei 70°C und erhielt eine viskose Lösung mit einem Feststoffgehalt von 39,8 % und einem K-Wert von 57.

Beispiel 4

60 g Methacrylsäure, 20 g Acrylamidomethylpropansulfonsäure und 280 g Wasser wurden gemischt und mit 50 %iger Natronlauge auf einen pH-Wert von 7 eingestellt. Danach wurden 533 g einer 60 %igen wässerigen Lösung von 3-Methyl-1-vinylimidazoliumchlorid zugegeben (Zulauf 1). In einer Rührapparatur mit Stickstoffspülung wurden 100 g Wasser, 96 g von Zulauf 1 und 5 g von Zulauf 2, bestehend aus 3 g 2,2'-Azobis(2-amidinopropan)dihydrochlorid und 60 g Wasser, vorgelegt. Man erhitzte auf 60°C und dosierte Zulauf 1 und 2 in jeweils 4 Stunden zu. Danach wurde die Temperatur auf 70°C erhöht und weitere 2 g 2,2'-Azo-bis(2-amidinopropan)dihydrochlorid in 75 ml Wasser zugegeben. Dann rührte man noch 1 Stunde bei 70°C und erhielt eine viskose Lösung mit einem Feststoffgehalt von 36,6 % und einem K-Wert von 53.

Beispiel 5

60 g Methacrylsäure und 200 g Wasser wurden gemischt und mit 50 %iger Natronlauge auf einen pH-Wert von 7 eingestellt. Danach wurden 755 g einer 60 %igen wässerigen Lösung von 3-Methyl-1-vinylimidazoliummethylsulfat zugegeben (Zulauf 1). In einer Rührapparatur mit Stickstoffspülung wurden 110 g Wasser, 107 g von Zulauf 1 und 6,3 g von Zulauf 2, bestehend aus 3 g 2,2'-Azo-bis(2-amidinopropan)dihydrochlorid und 60 g Wasser, vorgelegt. Man erhitzte auf 60°C und dosierte Zulauf 1 und 2 in jeweils 4 Stunden zu. Danach wurde die Temperatur auf 70°C erhöht und weitere 2 g 2,2'-Azo-bis(2-amidinopropan)dihydrochlorid in 60 ml Wasser zugegeben. Dann rührte man noch 1 Stunde bei 70°C und erhielt eine viskose Lösung mit einem Feststoffgehalt von 33,0 % und einem K-Wert von 42.

B) Verwendung der Polymere als Konditionierungsmittel

Beispiele 6 bis 8

Im folgenden wurden 3 Shampoos gemäß untenstehender Formulierung unter Verwendung der Polymere aus den Beispielen 1 bis 4 hergestellt und ihre haarkosmetischen Eigenschaften ermittelt.

Beispiele 9 bis 10 (Vergleichsversuche)

Es wurden 3 Shampoos gemäß untenstehender Formulierung hergestellt, wobei jedoch marktübliche kationische Polymere eingesetzt wurden.

Shampoo-Formulierung für Beispiele 6 bis 10 in Tabelle 1:

| | |
|---|---|
| Natriumlaurylethersulfat | 10,0 Gew.-% |
| Cocamidopropylbetain | 4,0 Gew.-% |
| Polymer (aus Beispiel 1,2,4 bzw. Vergleich) | 0,5 Gew.-% |
| Wasser | ad 100 Gew.-% |

Testmethoden:

a) Naßkämmbarkeit

An einer Zug/Druck-Prüfmaschine wird die Kämmarbeit bestimmt, welche notwendig ist, einen Kamm durch eine Haartresse zu ziehen. Die Kämmkraftabnahme wird wie folgt berechnet (je größer der Wert, desto besser das Shampoo).

$$\% \text{ Kämmkraftabnahme} = 100\ (1\ Ay/Ao)$$

Ay = Kämmarbeit nach Behandlung mit Testshampoo (siehe Beispiele)
Ao = Kämmarbeit nach Behandlung mit Shampoo ohne Polymer (Blindwert)

b) Schaumcremigkeit

Subjektive Bewertung mit einer Notenskala von 1 (sehr gut) bis 3 (schwach).

Sowohl die Kämmbarkeit des Haares als auch die Schaumcremigkeit wird durch die Art und Menge des

Polymers beeinflußt.

c) Stabilität der Shampoo-Formulierungen

Shampoo-Formulierungen nach oben genannter Zusammensetzung wurden hinsichtlich ihrer Stabilität geprüft. Die Polymere der Beispiele 1 bis 5 bilden nach Einrühren in die Tensidmischung weiße stabile Dispersionen, die auch nach mehreren Monaten keine Niederschläge aufweisen.

Tabelle 1:

| Anwendungstechnische Prüfungen mit dem oben genannten Testshampoo | | | |
|---|---|---|---|
| Shampoo Beispiel Nr. | Herstellbeispiel Nr. bzw. Handelsprodukt (Vergleich) | Kämmkraftabnahme 0,5 % Polymer | Schaumcremigkeit (Note) |
| 6 | 1 | 49 % | 1 |
| 7 | 2 | 39 % | 1 |
| 8 | 4 | 48 % | 1 |
| 9 | Polyquaternium-7 | 24 % | 2 |
| 10 | Polyquaternium-10 | 29 % | 2 |

Die Beispiele 6 bis 8 zeigen deutlich die hervorragenden Eigenschaften der Zusammensetzungen mit erfindungsgemäß zu verwendenden Polymeren gegenüber bekannten Zusammensetzungen (Beispiel 9 und 10).

**Patentansprüche**

**1.** Verwendung von kationischen Polymeren, erhältlich durch radikalisch initiierte Copolymerisation von

(a) 60 bis 99 Gew.-%, bezogen auf die Gesamtmenge aller Monomere, eines gegebenenfalls substituierten und gegebenenfalls quaternisierten 1-Vinylimidazols,

(b) 1 bis 40 Gew.-% einer eine polymerisierbare Doppelbindung enthaltenden Säure oder deren Salze und

(c) 0 bis 30 Gew.-% eines weiteren radikalisch copolymerisierbaren Monomeren, Gewichtsprozente jeweils bezogen auf die Gesamtmenge aller Monomere,

und Quaternisierung des Polymeren, sofern als Monomer (a) ein nicht quaterniertes 1-Vinylimidazol eingesetzt wird,
als Wirkstoffe in haarkosmetischen Zubereitungen.

**2.** Verwendung von kationischen Copolymeren gemäß Anspruch 1 mit einem K-Wert, gemessen 1%ig in 0,5 molarer Kochsalzlösung, von 10 bis 200, erhältlich durch radikalisch initiierte Copolymerisation eines Gemisches von

(a) 60 bis 99 Gew.-%, eines 1-Vinylimidazols der Formel I

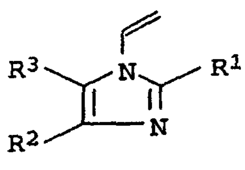

I,

in der $R^1$, $R^2$ und $R^3$ unabhängig voneinander Wasserstoff, Alkylreste mit 1 bis 4 C-Atomen oder Phenyl bedeuten oder 1-Vinylimidazole, die durch Anlagerung von Alkylresten mit 1 bis 24 C-Atomen, Aralkylresten mit 7 bis 30 C-Atomen oder mit Hydroxyalkylresten mit 2 bis 24 C-Atomen quaterniert sind,

(b) 1 bis 40 Gew.-% einer polymerisierbare Doppelbindungen enthaltenden Säure oder deren Salze und

(c) 0 bis 30 Gew.-% eines weiteren radikalisch copolymerisierbaren Monomeren und anschließende Quaternisierung des Polymeren, sofern als Monomeres (a) ein nicht quaternisiertes 1-Vinylimidazol eingesetzt wurde, durch Anlagerung einer Alkylgruppe mit 1 bis 24 C-Atomen, einer Hydroxyalkylgruppe mit 2 bis 24 C-Atomen oder einer Aralkylgruppe mit 7 bis 30 C-Atomen, als Wirkstoffe in haarkosmetischen Zubereitungen.

**3.** Verwendung von kationischen Copolymeren gemäß Anspruch 1, mit einem K-Wert, gemessen 1%ig in 0,5 molarer Kochsalzlösung, von 20 bis 150, erhältlich durch radikalisch initiierte Copolymerisation eines Gemisches von

(a) 65 bis 95 Gew.-% eines 1-Vinylimidazols der Formel I gemäß Anspruch 2,

(b) 5 bis 35 Gew.-% einer eine polymerisierbare Doppelbindung enthaltenden Säure mit 2 bis 8 C-Atomen ausgewählt aus der Gruppe bestehend aus

ba) $\alpha,\beta$-ungesättigten Carbonsäuren,
bb) ungesättigten Sulfonsäuren und
bc) ungesättigten Phosphonsäuren

und

(c) 0 bis 20 Gew.-% eines weiteren radikalisch copolymerisierbaren Monomeren

und Quaternisierung der Copolymere mit Alkylhalogeniden oder Dialkylsulfaten mit 1 bis 24 C-Atomen, Aralkylhalogeniden mit 7 bis 30 C-Atomen oder Alkylenoxiden mit 2 bis 24 C-Atomen.

**4.** Verwendung von kationischen Copolymeren gemäß Anspruch 1, **dadurch gekennzeichnet, daß** die Copolymere den Bestandteil

(a) zu 70 bis 90 Gew.-%, den Bestandteil
(b) zu 10 bis 30 Gew.-% und den Bestandteil
(c) zu 0 bis 10 Gew.-% enthalten.

**5.** Verwendung von kationischen Copolymeren gemäß Anspruch 1, **dadurch gekennzeichnet, daß** der Bestandteil

(a) 1-Vinylimidazol oder 2-Methyl-1-vinylimidazol und als Bestandteil
(b) Acrylsäure, Methacrylsäure oder Acrylamidomethylpropansulfonsäure

ist und die Quaternisierung mit Methylchlorid, Dimethylsulfat oder Diethylsulfat durchgeführt worden ist.

**6.** Verwendung von kationischen Copolymeren gemäß Anspruch 1 als Konditioniermittel in Shampoos.

**7.** Shampoos enthaltend an sich bekannte Detergentien und als Konditioniermittel 0,01 bis 5 Gew.-%, bezogen auf das fertige Shampoo, an kationischen Copolymeren, erhältlich durch radikalisch initiierte Copolymerisation von

(a) 60 bis 99 Gew.-%, bezogen auf die Gesamtmenge aller Monomere, eines gegebenenfalls substituierten und gegebenenfalls quaternisierten 1-Vinylimidazols,

(b) 1 bis 40 Gew.-% einer eine polymerisierbare Doppelbindung enthaltenden Säure oder deren Salze und

(c) 0 bis 30 Gew.-% eines weiteren radikalisch copolymerisierbaren Monomeren, Gewichtsprozente jeweils, bezogen auf die Gesamtmenge aller Monomere,

und Quaternisierung des Polymeren, sofern als Monomer (a) ein nicht quaterniertes 1-Vinylimidazol eingesetzt wird.

**8.** Shampoos gemäß Anspruch 7, enthaltend als Konditioniermittel kationische Copolymere mit einem K-Wert, gemessen 1%ig in 0,5 molarer Kochsalzlösung, von 10 bis 200, erhältlich durch radikalisch initiierte Copolymerisation

eines Gemisches von

(a) 60 bis 99 Gew.-% eines 1-Vinylimidazols der Formel I

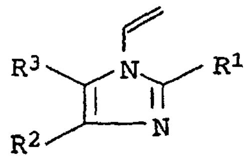

I,

in der $R^1$, $R^2$ und $R^3$ unabhängig voneinander Wasserstoff, Alkylreste mit 1 bis 4 C-Atomen oder Phenyl bedeuten und das gegebenenfalls durch Anlagerung von Alkylresten mit 1 bis 24 C-Atomen, Aralkylresten mit 7 bis 30 C-Atomen oder mit Hydroxyalkylresten mit 2 bis 24 C-Atomen quaternisiert ist,

(b) 1 bis 40 Gew.-% einer polymerisierbare Doppelbindungen enthaltenden Säure oder deren Salze und

(c) 0 bis 30 Gew.-& eines weiteren radikalisch copolymerisierbaren Monomeren

und anschließende Quaternisierung des Polymeren, sofern als Monomeres (a) ein nicht quaternisiertes 1-Vinylimidazol eingesetzt wurde, durch Anlagerung einer Alkylgruppe mit 1 bis 24 C-Atomen, einer Hydroxyalkylgruppe mit 2 bis 24 C-Atomen oder einer Aralkylgruppe mit 7 bis 30 C-Atomen.

**9.** Shampoos gemäß Anspruch 7, enthaltend als Konditioniermittel kationische Polymere mit einem K-Wert, gemessen 1%ig in 0,5 molarer Kochsalzlösung, von 20 bis 150, erhältlich durch radikalisch initiierte Copolymerisation eines Gemisches von

(a) 65 bis 95 Gew.-% eines 1-Vinylimidazols der Formel I gemäß Anspruch 2,

(b) 5 bis 35 Gew.-% einer eine polymerisierbare Doppelbindung enthaltenden Säure mit 2 bis 8 C-Atomen ausgewählt aus der Gruppe bestehend aus

(ba) $\alpha,\beta$-ungesättigten Carbonsäuren,
(bb) ungesättigten Sulfonsäuren und
(bc) ungesättigten Phosphonsäuren

und

(c) 0 bis 20 Gew.-% eines weiteren radikalisch copolymerisierbaren Monomeren

und Quaternisierung der Copolymere mit Alkylhalogeniden oder Dialkylsulfaten mit 1 bis 24 C-Atomen, Aralkylhalogeniden mit 7 bis 30 C-Atomen oder Alkylenoxiden mit 2 bis 24 C-Atomen.

**10.** Shampoos gemäß Anspruch 7, enthaltend als Konditionierungsmittel kationische Polymere, **dadurch gekennzeichnet, daß** der Bestandteil

(a) 1-Vinylimidazol oder 2-Methyl-1-vinylimidazol und der Bestandteil

(b) Acrylsäure, Methacrylsäure oder Acrylamidomethylpropansulfonsäure

ist und die Quaternisierung mit Methylchlorid, Dimethylsulfat oder Diethylsulfat durchgeführt worden ist.

## Claims

**1.** The use of cationic polymers obtainable by free-radically initiated copolymerization of

(a) from 60 to 99% by weight, based on the overall amount of all monomers, of a substituted or unsubstituted and quaternized or nonquaternized 1-vinylimidazole,

(b) from 1 to 40% by weight of an acid which contains a polymerizable double bond, or of salts of such an acid, and

(c) from 0 to 30% by weight of a further free-radically copolymerizable monomer, percentages by weight being based in each case on the overall amount of all monomers,

and quaternization of the polymer if a nonquaternized 1-vinylimidazole was employed as monomer a) as active ingredients in cosmetic hair formulations.

**2.** The use of cationic copolymers as claimed in claim 1 having a K value, measured as a 1% strength solution in 0.5 molar sodium chloride solution, of from 10 to 200, obtainable by free-radically initiated copolymerization of a mixture of

(a) from 60 to 99% by weight of a 1-vinylimidazol of the formula I

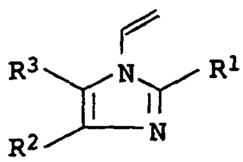

I,

in which $R^1$, $R^2$ and $R^3$ independently are hydrogen, alkyls of 1 to 4 carbons or phenyl, or of 1-vinylimidazoles quaternized by adding on alkyl radicals of 1 to 24 carbons, aralkyl radicals of 7 to 30 carbons or hydroxyalkyl radicals of 2 to 24 carbons,

(b) from 1 to 40% by weight of an acid containing polymerizable double bonds, or of salts of such an acid, and

(c) from 0 to 30% by weight of a further free-radically copolymerizable monomer, and subsequent quaternization of the polymer if a nonquaternized 1-vinylimidazole was employed as monomer a), by adding on an alkyl of 1 to 24 carbons, a hydroxyalkyl of 2 to 24 carbons or an aralkyl of 7 to 30 carbons, as active ingredients in cosmetic hair formulations.

**3.** The use of cationic copolymers as claimed in claim 1 having a K value, measured as a 1% strength solution in 0.5 molar sodium chloride solution, from 20 to 150, obtainable by free-radically initiated copolymerization of a mixture of

(a) from 65 to 95% by weight of a 1-vinylimidazole of the formula I as set forth in claim 2,

(b) from 5 to 35% by weight of an acid which contains a polymerizable double bond and has 2 to 8 carbon atoms, selected from the group consisting of

ba) $\alpha,\beta$-unsaturated carboxylic acids,
bb) unsaturated sulfonic acids and
bc) unsaturated phosphonic acids,

and

(c) from 0 to 20% by weight of a further free-radically copolymerizable monomer

and quaternization of the copolymers with alkyl halides or dialkyl sulfates of 1 to 24 carbons, aralkyl halides of 7 to 30 carbons or alkylene oxides of 2 to 24 carbons.

**4.** The use of cationic copolymers as claimed in claim 1, wherein the copolymers comprise from 70 to 90% by weight of constituent (a),

from 10 to 30% by weight of constituent (b) and
from 0 to 10% by weight of constituent (c).

**5.** The use of cationic copolymers as claimed in claim 1, wherein the constituent

(a) is 1-vinylimidazole or 2-methyl-1-vinylimidazole and the constituent
(b) is acrylic, methacrylic or acrylamidomethylpropanesulfonic acid
and the quaternization has been carried out with methyl chloride, dimethyl sulfate or diethyl sulfate.

**6.** The use of cationic copolymers as claimed in claim 1 as conditioning agents in shampoos.

**7.** A shampoo comprising conventional detergents and, as conditioning agent, from 0.01 to 5% by weight, based on the finished shampoo, of cationic copolymers obtainable by free-radically initiated copolymerization of

(a) from 60 to 99% by weight, based on the overall amount of all monomers, of a substituted or unsubstituted and quaternized or nonquaternized 1-vinylimidazole,

(b) from 1 to 40% by weight of an acid which contains a polymerizable double bond, or of salts of such an acid, and

(c) from 0 to 30% by weight of a further free-radically copolymerizable monomer, percentages by weight being based in each case on the overall amount of all monomers,

and quaternization of the polymer if a nonquaternized 1-vinylimidazole was employed as monomer a).

**8.** A shampoo as claimed in claim 7, comprising as conditioning agent cationic copolymers having a K value, measured as a 1% strength solution in 0.5 molar sodium chloride solution, of from 10 to 200, obtainable by free-radically initiated copolymerization of a mixture of

(a) from 60 to 99% by weight of a 1-vinylimidazole of the formula I

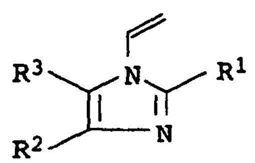

I,

in which $R^1$, $R^2$ and $R^3$ independently are hydrogen, alkyls of 1 to 4 carbons or phenyl, and which 1-vinylimidazole is quaternized, if appropriate, by adding on alkyl radicals of 1 to 24 carbons, aralkyl radicals of 7 to 30 carbons or hydroxyalkyl radicals of 2 to 24 carbons,

(b) from 1 to 40% by weight of an acid containing polymerizable double bonds, or of salts of such an acid, and

(c) from 0 to 30% by weight of a further free-radically copolymerizable monomer,

and subsequent quaternization of the polymer if a nonquaternized 1-vinylimidazole was employed as monomer a), by adding on an alkyl of 1 to 24 carbons, a hydroxyalkyl of 2 to 24 carbons or an aralkyl of 7 to 30 carbons.

**9.** A shampoo as claimed in claim 7 comprising as conditioning agent cationic polymers having a K value, measured as a 1% strength solution in 0.5 molar sodium chloride solution, from 20 to 150, obtainable by free-radically initiated copolymerization of a mixture of

(a) from 65 to 95% by weight of a 1-vinylimidazole of the formula I as set forth in claim 2,

(b) from 5 to 35% by weight of an acid which contains a polymerizable double bond and has 2 to 8 carbon atoms, selected from the group consisting of

ba) α,β-unsaturated carboxylic acids,
bb) unsaturated sulfonic acids and
bc) unsaturated phosphonic acids,

and

(c) from 0 to 20% by weight of a further free-radically copolymerizable monomer

and quaternization of the copolymers with alkyl halides or dialkyl sulfates of 1 to 24 carbons, aralkyl halides of 7 to 30 carbons or alkylene oxides of 2 to 24 carbons.

**10.** A shampoo as claimed in claim 7 comprising as conditioning agent cationic copolymers, wherein the constituent

(a) is 1-vinylimidazole or 2-methyl-1-vinylimidazole and the constituent
(b) is acrylic, methacrylic or acrylamidomethylpropanesulfonic acid

and the quaternization has been carried out with methyl chloride, dimethyl sulfate or diethyl sulfate.

## Revendications

**1.** Utilisation de polymères cationiques, pouvant être obtenus par copolymérisation initiée par voie radicalaire de

(a) 60 à 99 % en poids, par rapport à la quantité totale de tous les monomères, d'un 1-vinylimidazole éventuellement substitué et éventuellement quaternisé,
(b) 1 à 40 % en poids d'un acide contenant une double liaison polymérisable ou de ses sels, et
(c) 0 à 30 % en poids d'un autre monomère copolymérisable par voie radicalaire, les pourcentages en poids étant à chaque fois par rapport à la quantité totale de tous les monomères,

et quaternisation des polymères, dans la mesure où on utilise comme monomère (a) un 1-vinylimidazole non-quaternisé,
comme substances actives dans des compositions cosmétiques capillaires.

**2.** Utilisation de copolymères cationiques selon la revendication 1 ayant un indice K, mesuré à 1 % dans une solution 0,5 molaire de chlorure de sodium, de 10 à 200, pouvant être obtenus par copolymérisation initiée par voie radicalaire d'un mélange de

(a) 60 à 99 % en poids d'un 1-vinylimidazole de formule I

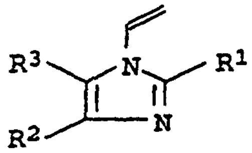

I,

dans laquelle $R^1$, $R^2$ et $R^3$ désignent, indépendamment l'un de l'autre, l'hydrogène, des restes alkyle ayant 1 à 4 atomes de carbone ou phényle, ou de groupes 1-vinylimidazole, qui sont quaternisés par fixation par addition de restes alkyle ayant 1 à 24 atomes de carbone, de restes aralkyle ayant 7 à 30 atomes de carbone ou avec des restes hydroxyalkyle ayant 2 à 24 atomes de carbone,
(b) 1 à 40 % en poids d'un acide contenant des doubles liaisons polymérisables ou ses sels et
(c) 0 à 30 % en poids d'un autre monomère copolymérisable par voie radicalaire

et ensuite quaternisation du polymère, dans la mesure où on a utilisé comme monomère (a) un 1-vinylimidazole non quaternisé, par fixation par fixation par addition d'un groupe alkyle ayant 1 à 24 atomes de carbone, d'un groupe hydroxyalkyle ayant 2 à 24 atomes de carbone ou d'un groupe aralkyle ayant 7 à 30 atomes de carbone, comme substances actives dans des compositions cosmétiques capillaires.

**3.** Utilisation de copolymères cationiques selon la revendication 1, ayant un indice K, mesuré à 1 % dans une solution 0,5 molaire de chlorure de sodium, de 20 à 150, pouvant être obtenus par copolymérisation initiée par voie radicalaire d'un mélange de

(a) 65 à 95 % en poids d'un 1-vinylimidazole de formule I selon la revendication 2,
(b) 5 à 35 % en poids d'un acide contenant une double liaison polymérisable ayant 2 à 8 atomes de carbone, choisi dans le groupe consistant en

ba) acides carboxyliques $\alpha,\beta$-insaturés,
bb) acides sulfoniques insaturés et
bc) acides phosphoniques insaturés

et
(c) 0 à 20 % en poids d'un autre monomère copolymérisable par voie radicalaire

et quaternisation des copolymères avec des halogénures d'alkyle ou des sulfates de dialkyle ayant 1 à 24 atomes de carbone, des halogénures d'aralkyle ayant 7 à 30 atomes de carbone ou des oxydes d'alkylène ayant 2 à 24 atomes de carbone.

**4.** Utilisation de copolymères cationiques selon la revendication 1, **caractérisée par le fait que** les copolymères contiennent
le composant (a) à raison de 70 à 90 % en poids,
le composant (b) à raison de 10 à 30 % en poids, et
le composant (c) à raison de 0 à 10 % en poids.

**5.** Utilisation de copolymères cationiques selon la revendication 1, **caractérisée par le fait que**
le composant (a) est le 1-vinylimidazole ou le 2-méthyl-1-vinylimidazole et
le composant (b) est l'acide acrylique, l'acide méthacrylique ou l'acide acrylamidométhylpropanesulfonique
et la quaternisation a été effectuée avec du chlorure de méthyle, du sulfate de diméthyle ou du sulfate de diéthyle.

**6.** Utilisation de copolymères cationiques selon la revendication 1 comme agent de conditionnement dans des shampooings.

**7.** Shampooings contenant des détergents connus en soi et, comme agent de conditionnement, 0,01 à 5 % en poids, par rapport au shampooing fini, de copolymères cationiques, pouvant être obtenus par copolymérisation initiée par voie radicalaire de

(a) 60 à 99 % en poids, par rapport à la quantité totale de tous les monomères, d'un 1-vinylimidazole éventuellement substitué et éventuellement quaternisé,
(b) 1 à 40 % en poids d'un acide contenant une double liaison polymérisable ou ses sels et
(c) 0 à 30 % en poids d'un autre monomère copolymérisable par voie radicalaire, les pourcentages en poids étant à chaque fois par rapport à la quantité totale de tous les monomères,

et quaternisation du polymère, dans la mesure où on utilise comme monomère (a) un 1-vinylimidazole non quaternisé.

**8.** Shampooings selon la revendication 7, contenant comme agent de conditionnement des copolymères cationiques ayant un indice K, mesuré à 1 % dans une solution 0,5 molaire de chlorure de sodium, de 10 à 200, pouvant être obtenus par copolymérisation initiée par voie radicalaire d'un mélange de

(a) 60 à 99 % en poids d'un 1-vinylimidazole de formule I

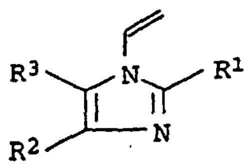

I,

dans laquelle $R^1$, $R^2$ et $R^3$ désignent, indépendamment l'un de l'autre, l'hydrogène, des restes alkyle ayant 1 à 4 atomes de carbone ou phényle, et qui est éventuellement quaternisé par fixation par addition éventuelle de restes alkyle ayant 1 à 24 atomes de carbone, de restes aralkyle ayant 7 à 30 atomes de carbone ou avec des restes hydroxyalkyle ayant 2 à 24 atomes de carbone,
(b) 1 à 40 % en poids d'un acide contenant des doubles liaisons polymérisables ou ses sels et
(c) 0 à 30 % en poids d'un autre monomère copolymérisable par voie radicalaire

et ensuite quaternisation du polymère, dans la mesure où on a utilisé comme monomère (a) un 1-vinylimidazole non quaternisé, par fixation par fixation par addition d'un groupe alkyle ayant 1 à 24 atomes de carbone, d'un groupe hydroxyalkyle ayant 2 à 24 atomes de carbone ou d'un groupe aralkyle ayant 7 à 30 atomes de carbone.

**9.** Shampooings selon la revendication 7, contenant comme agent de conditionnement des polymères cationiques ayant un indice K, mesuré à 1 % dans une solution 0,5 molaire de chlorure de sodium, de 20 à 150, pouvant être obtenus par copolymérisation initiée par voie radicalaire d'un mélange de

(a) 65 à 95 % en poids d'un 1-vinylimidazole de formule I selon la revendication 2,
(b) 5 à 35 % en poids d'un acide contenant une double liaison polymérisable ayant 2 à 8 atomes de carbone, choisi dans le groupe consistant en

ba) acides carboxyliques $\alpha,\beta$-insaturés,
bb) acides sulfoniques insaturés et
bc) acides phosphoniques insaturés

et
(c) 0 à 20 % en poids d'un autre monomère copolymérisable par voie radicalaire et quaternisation des copolymères avec des halogénures d'alkyle ou des sulfates de dialkyle ayant 1 à 24 atomes de carbone, des halogénures d'aralkyle ayant 7 à 30 atomes de carbone ou des oxydes d'alkylène ayant 2 à 24 atomes de carbone.

**10.** Shampooings selon la revendication 7, contenant comme agent de conditionnement des polymères cationiques, **caractérisés par le fait que**
le composant (a) est le 1-vinylimidazole ou le 2-méthylvinylimidazole et
le composant (b) est l'acide acrylique, l'acide méthacrylique ou l'acide acrylamidométhylpropanesulfonique
et la quaternisation a été effectuée avec du chlorure de méthyle, du sulfate de diméthyle ou du sulfate de diéthyle.